(19) **Europäisches Patentamt**
**European Patent Office**
**Office européen des brevets**

(11) **EP 1 919 537 B1**

(12) **FASCICULE DE BREVET EUROPEEN**

(45) Date de publication et mention
de la délivrance du brevet:
**18.04.2012 Bulletin 2012/16**

(51) Int Cl.:
*A61M 5/178* *(2006.01)*  *A61M 5/28* *(2006.01)*
*A61L 2/07* *(2006.01)*  *A61L 2/20* *(2006.01)*

(21) Numéro de dépôt: **06794370.4**

(22) Date de dépôt: **30.08.2006**

(86) Numéro de dépôt international:
**PCT/FR2006/002007**

(87) Numéro de publication internationale:
**WO 2007/028876 (15.03.2007 Gazette 2007/11)**

(54) **SERINGUE DESTINEE A ETRE PRE-REMPLIE PUIS STERILISEE PAR AUTOCLAVAGE A LA VAPEUR**

VORFÜLLBARE UND DANN IM DAMPFAUTOKLAVEN STERILISIERBARE SPRITZE

SYRINGE DESIGNED TO BE PRE-FILLED THEN STERILIZED BY STEAM AUTOCLAVING

(84) Etats contractants désignés:
**AT BE BG CH CY CZ DE DK EE ES FI FR GB GR HU IE IS IT LI LT LU LV MC NL PL PT RO SE SI SK TR**

(30) Priorité: **02.09.2005 FR 0509011**

(43) Date de publication de la demande:
**14.05.2008 Bulletin 2008/20**

(73) Titulaire: **Laboratoire Aguettant**
**69353 Lyon Cedex 07 (FR)**

(72) Inventeur: **FREZZA, Pierre**
**F-69390 Charly (FR)**

(74) Mandataire: **Maureau, Philippe et al**
**Cabinet GERMAIN & MAUREAU**
**12, rue Boileau**
**B.P. 6153**
**69466 Lyon Cedex 06 (FR)**

(56) Documents cités:
**EP-A- 0 599 649  WO-A-96/30066**
**US-A- 3 468 471**

**Description**

La présente invention concerne une seringue, en particulier une seringue destinée à être pré-remplie puis stérilisée par autoclavage à la vapeur, ainsi qu'un ensemble comportant un emballage et une telle seringue conditionnée dans cet emballage.

Une seringue dite "pré-remplie" est un produit prêt à l'emploi, non réutilisable. Une seringue de ce type est remplie du liquide souhaité et stérilisée industriellement par le laboratoire pharmaceutique. La stérilité de l'ensemble contenant/contenu est obtenue soit par remplissage en aseptique de seringues dont les composants ont étés préalablement stérilisés, soit par une stérilisation finale à la vapeur de l'ensemble contenant/contenu. La présente invention ne concerne que ce dernier mode de stérilisation.

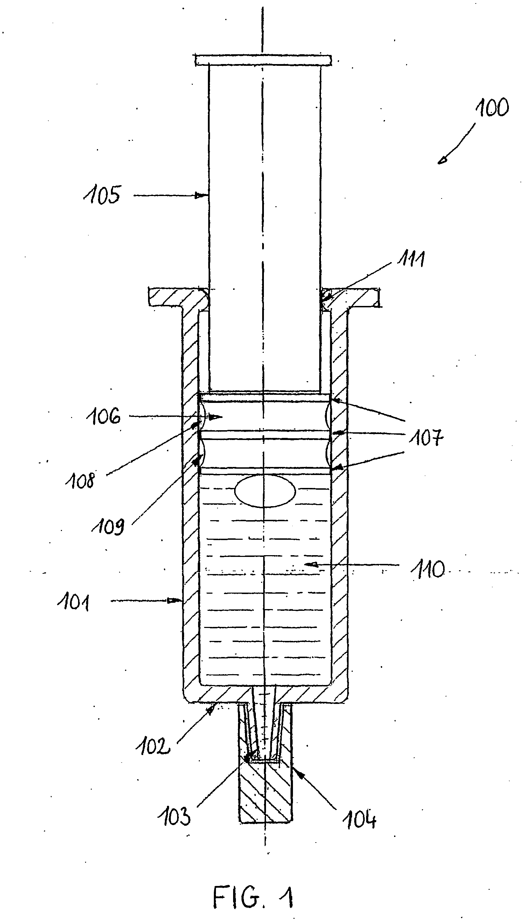

Une seringue pré-remplie de l'art antérieur est représentée en coupe longitudinale sur la figure 1.

La seringue 100 comporte un corps 101 cylindrique fermé à son extrémité aval par une paroi 102 comportant un accès 103 de type « Luer » ou « Luer-lock », lui même fermé de manière étanche par un bouchon amovible 104. L'extrémité amont du corps 101 est ouverte pour permettre l'introduction en force d'une tige 105, formant poussoir, munie d'un piston 106 formant joint d'étanchéité, qui comporte généralement trois lèvres d'étanchéité 107 définissant deux chambres annulaires étanches 108, 109.

L'ensemble tige 105 - piston 106 peut coulisser de manière étanche dans le corps 101 sous l'action de la poussée exercée par exemple par une infirmière, chassant ainsi le liquide 110 contenu dans le corps 101 vers un patient via l'accès 103.

Enfin, l'extrémité amont du corps 101 comporte un bourrelet annulaire 111 destiné à servir de butée à l'ensemble tige 105 - piston 106 pour l'empêcher de ressortir du corps 101.

Le procédé industriel de remplissage et stérilisation de la seringue 100 est le suivant. Après mise en place du bouchon 104, remplissage, mise en place du piston 106 puis de la tige 105 par vissage sur le piston 106, la seringue 100 est conditionnée dans un emballage (« blister ») comportant une partie en matière plastique thermoformée fermée par un opercule en papier pelable. Ce papier possède la particularité d'être perméable à la vapeur d'eau mais d'être une barrière sensiblement étanche aux microorganismes.

La stérilisation à la vapeur, pour satisfaire aux exigences réglementaires (garantie de stérilité même en cas de forte contamination initiale), doit être effectuée à une température d'au moins 121 °C pendant au moins 15 minutes en chaleur dite « humide », c'est à dire que toutes les parties nécessitant d'être stérilisées doivent être en contact avec la vapeur, que cette dernière provienne de l'enceinte de l'autoclave après avoir traversé l'opercule de l'emballage, ou du contenu vaporisé de la seringue.

Or, les chambres annulaires 108, 109 ménagées entre les lèvres 107 du piston 106 étant étanches, elles sont non accessibles à la vapeur. La stérilisation s'effectuera donc en chaleur dite « sèche ». En conséquence, afin de garantir la stérilité de ces chambres annulaires, il est nécessaire que la durée de stérilisation soit beaucoup plus longue qu'en chaleur « humide » (de l'ordre de 60 minutes environ, au lieu de 15 minutes). Ce cycle de stérilisation rallongé présente trois inconvénients majeurs :

- coût de production nettement plus élevé ;
- dégradation plus importante de la matière constitutive du corps de la seringue, augmentant le risque de relargage de produits de dégradation (additifs plastiques ou composants du verre tel que l'aluminium) dans le liquide 110 ;
- impossibilité de stériliser à 121 °C pendant 60 minutes certains principes actifs car ils subiraient une dégradation rédhibitoire.

Une autre seringue connue dans l'art antérieur est divulguée dans le document EP-A-0599649. Le préambule de la revendication 1 est rédigé d'après ce document.

La présente invention tel que revendiquée vise à remédier aux inconvénients mentionnés ci-dessus, de manière simple et sans surcoût important pour la seringue.

A cet effet, et selon un premier aspect, l'invention concerne une seringue comprenant :

- un corps comportant une paroi latérale sensiblement cylindrique présentant une extrémité amont ouverte au voisinage de laquelle est ménagé un bourrelet annulaire intérieur et une extrémité aval fermée par une paroi transversale munie d'un orifice ;
- une tige comportant à une extrémité un piston formant joint d'étanchéité et possédant au moins deux lèvres d'étanchéité annulaires entre lesquelles est définie au moins une chambre annulaire, ladite tige étant apte à être introduite et à coulisser dans le corps.

Une chambre intérieure destinée à être au moins partiellement remplie d'un contenu est alors définie entre le piston et la paroi transversale du corps.

Selon une définition générale de l'invention, la seringue comprend en outre des moyens de passage ménagés dans le corps de la seringue et agencés pour mettre en communication ladite chambre annulaire ou lesdites chambres annulaires du piston avec l'extérieur du corps ou avec la chambre intérieure du corps, lorsque le piston est situé à l'intérieur du corps en butée contre le bourrelet annulaire.

En pratique, en position de stockage (c'est-à-dire lorsque la seringue est pré-remplie, et à température de stockage), les moyens de passage sont situés en amont du piston et l'étanchéité du contenu de la chambre intérieure du corps de la seringue n'est pas remise en question.

Lors de la stérilisation, du fait de la pression à l'intérieur du corps due au passage du contenu de la seringue en phase vapeur, le piston vient en butée contre le bourrelet. Grâce aux moyens de passage, les chambres annulaires du piston sont alors accessibles à la vapeur, provenant soit de l'extérieur du corps (à savoir de l'enceinte de l'autoclave où s'effectue la stérilisation) soit de l'intérieur même du corps, c'est-à-dire du contenu passé en phase vapeur. Bien entendu, une même chambre ne peut être en communication à la fois avec l'extérieur du corps et avec la chambre intérieure, sous peine de perte d'étanchéité de la chambre intérieure.

L'invention permet donc la stérilisation par chaleur humide entre les lèvres du piston de la seringue, et de ce fait de diminuer fortement la quantité de chaleur nécessaire à la stérilisation.

Avantageusement, les moyens de passage sont agencés pour mettre également en communication au moins une zone de la face extérieure d'au moins une lèvre d'étanchéité avec l'extérieur du corps ou avec la chambre intérieure du corps, lorsque le piston est en butée contre le bourrelet annulaire, au moins une autre lèvre d'étanchéité continuant, dans cette position du piston, à assurer l'étanchéité de la chambre intérieure du corps.

Par « étanchéité de la chambre intérieure », on entend l'absence d'entrée d'un quelconque produit dans ladite chambre intérieure et l'absence de fuite de ce contenu hors du corps de la seringue (le contenu pouvant néanmoins atteindre les chambres annulaires du piston).

Dans toute la description, on utilisera les termes «amont» et « aval », définis par rapport au sens d'écoulement du contenu hors de la seringue, lors de l'utilisation de celle-ci.

Selon un premier mode de réalisation, les moyens de passage comprennent au moins une rainure ménagée sensiblement axialement dans la paroi latérale du corps cylindrique, dans sa face intérieure, ladite rainure débouchant hors du corps à l'extrémité amont de celui-ci et s'étendant jusqu'à une extrémité aval située, lorsque le piston est en butée contre le bourrelet annulaire intérieur, en amont de la lèvre d'étanchéité aval et en aval de la lèvre d'étanchéité située immédiatement en amont de la lèvre d'étanchéité aval, la profondeur radiale de la rainure étant suffisante pour rompre localement l'étanchéité entre la face extérieure d'au moins la lèvre d'étanchéité amont et la face intérieure de la paroi latérale du corps.

Selon un deuxième mode de réalisation, les moyens de passage comprennent au moins un orifice pratiqué dans la paroi latérale du corps et agencé pour mettre en communication la chambre annulaire ou les chambres annulaires du piston avec l'extérieur du corps lorsque le piston est en butée contre le bourrelet annulaire, le bord aval dudit orifice étant situé, lorsque le piston est en butée contre le bourrelet annulaire intérieur, en amont de la lèvre d'étanchéité aval et en aval de la lèvre d'étanchéité située immédiatement en amont de la lèvre d'étanchéité aval.

Selon un troisième mode de réalisation, les moyens de passage comprennent au moins une gorge ménagée dans la paroi latérale du corps cylindrique, dans sa face intérieure, ladite gorge s'étendant entre:

- une extrémité amont située, lorsque le piston est en butée contre le bourrelet annulaire intérieur, en aval de la lèvre d'étanchéité amont et en amont de la lèvre d'étanchéité située immédiatement en aval de la lèvre d'étanchéité amont;
- et une extrémité aval située, lorsque le piston est en butée contre le bourrelet annulaire intérieur, en aval de la lèvre d'étanchéité aval ;

la longueur axiale de la gorge étant inférieure à la longueur axiale totale du piston et la profondeur radiale de la gorge étant suffisante pour rompre localement l'étanchéité entre la face extérieure d'au moins la lèvre d'étanchéité aval et la face intérieure de la paroi latérale du corps.

La gorge est par exemple annulaire, de même axe que la paroi latérale cylindrique du corps.

Selon une réalisation possible, le piston possède trois lèvres d'étanchéité annulaires définissant deux chambres annulaires distinctes, les moyens de passage étant agencés pour mettre en communication chacune des chambres annulaires avec l'extérieur du corps ou avec la chambre intérieure du corps lorsque le piston est situé à l'intérieur du corps en butée contre le bourrelet annulaire.

Enfin, selon un deuxième aspect, l'invention concerne un ensemble comportant d'une part un emballage sensiblement imperméable aux bactéries dont au moins une partie est perméable à la vapeur d'eau et d'autre part une seringue tel que précédemment décrite, ladite seringue étant conditionnée dans ledit emballage et sa chambre intérieure au moins partiellement remplie d'un contenu.

On décrit à présent, à titre d'exemples non limitatifs, plusieurs formes de réalisation possibles de l'invention, en référence aux figures annexées :

La figure 1 est une vue en coupe longitudinale d'une seringue de l'art antérieur ;

Les figures 2 et 3 sont des vues partielles en coupe longitudinale d'une seringue pré-remplie selon un premier mode de réalisation de l'invention, respectivement lors du stockage et lors de la stérilisation ;

Les figures 4 et 5 sont des vues analogues aux figures 2 et 3, illustrant un deuxième mode de réalisation ; et

Les figures 6 et 7 sont des vues analogues aux figures 2 et 3, illustrant un troisième mode de réalisation, le corps de la seringue étant représenté dans son intégralité.

Une seringue 1 comprend tout d'abord un corps 2 comportant une paroi latérale 3 sensiblement cylindrique d'axe 4. La paroi latérale 3 présente une extrémité amont ouverte et une extrémité aval fermée par une paroi transversale 5 munie d'un orifice 6 et prolongée par un embout conique 7 de type « Luer » ou « Luer lock ».

A son extrémité amont, le corps comporte d'une part une collerette 8 destinée à servir d'appui aux doigts d'une infirmière, et d'autre part un bourrelet annulaire intérieur 9.

La seringue 1 comprend également une tige 10 formant poussoir qui comporte à son extrémité aval un piston 11. Le piston 11 possède trois lèvres d'étanchéité annulaires, respectivement une lèvre amont 12, une lèvre intermédiaire 13 et une lèvre aval 14, destinées à coopérer avec la face intérieure 15 de la paroi latérale 3 du corps 2. Entre deux lèvres successives est définie une chambre annulaire. Dans la réalisation représentée, le piston 11 comporte donc deux chambres annulaires 16, 17.

La seringue 1 (corps et tige) est ici réalisée en matière plastique, mais elle pourrait être en verre.

La tige 10 est apte à être introduite dans le corps 2 et à y coulisser de manière étanche, sous l'action de poussée d'un utilisateur. Le piston 11 et l'intérieur du corps 2 sont généralement siliconés afin de faciliter le coulissement du piston.

Est ainsi définie une chambre intérieure dans le corps 2, entre la paroi transversale 5 et le piston 11. La chambre intérieure est remplie d'un contenu 18 qui peut être une solution médicamenteuse, un solvant, etc. Il subsiste aussi généralement dans cette chambre intérieure une bulle de gaz 19 (air ou azote par exemple, selon les cas).

Enfin, la seringue 1 comporte un bouchon 20 amovible apte à obturer l'orifice 6 ménagé dans la paroi transversale 5 du corps 2.

La seringue 1, munie du bouchon 20, pré-remplie et équipée de la tige 10, est placée dans un emballage du type précédemment décrit, puis l'ensemble est introduit dans un autoclave pour la stérilisation à la vapeur de la seringue 1.

Selon l'invention, des moyens de passage sont ménagés dans le corps 2 de la seringue 1 pour permettre la stérilisation des chambres annulaires 16,17 du piston 11 par de la vapeur:

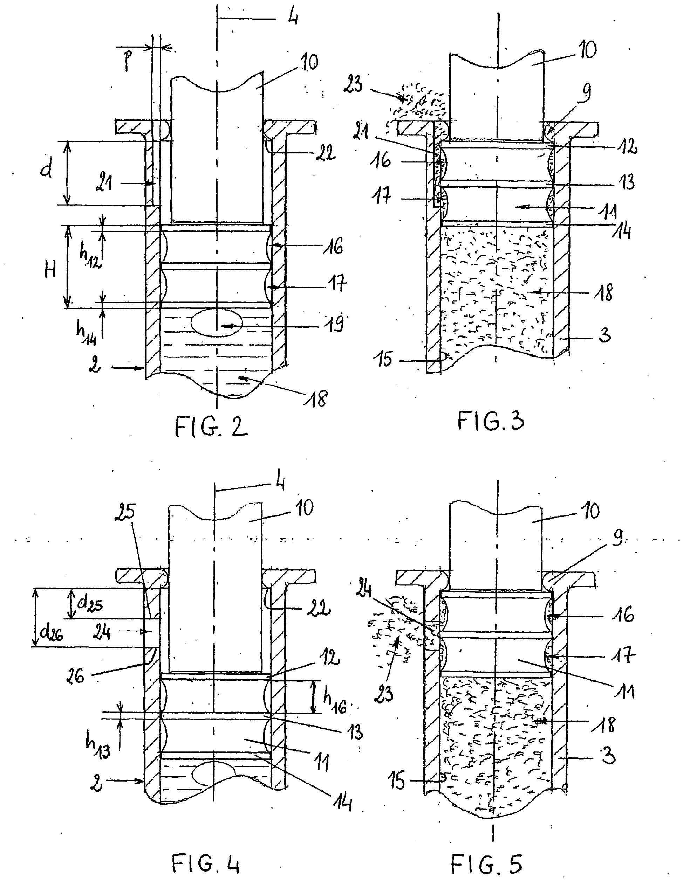

Selon un premier mode de réalisation, représenté sur les figures 2 et 3, les moyens de passage sont constitués par au moins une rainure 21 ménagée sensiblement axialement dans la paroi latérale 3 du corps 2, depuis la face intérieure 15. De préférence, la rainure 21 débouche hors du corps 2 à l'extrémité amont de celui-ci, en interrompant localement le bourrelet 9. En variante, la (ou les) rainure 21 pourrait ne pas déboucher à l'extérieur du corps, mais posséder une extrémité amont située au voisinage de la face aval 22 du bourrelet 9.

La rainure 21, ou chacune des rainures 21, possède les caractéristiques suivantes :

-    la distance axiale d entre la face aval 22 du bourrelet 9 et l'extrémité aval de la rainure 21 est telle que :

$$d > h_{12} + h_{16} + h_{13} \quad et \quad d < H - h_{14,}$$

où - H est la longueur axiale totale du piston 11,

-    $h_{12}$, $h_{13}$ et $h_{14}$ sont respectivement la longueur axiale de la lèvre d'étanchéité amont 12, intermédiaire 13 et aval 14 du piston 11,
-    $h_{16}$ est la longueur axiale de la chambre annulaire amont 16 du piston 11 ;

-    la profondeur radiale p de la rainure 21 est suffisante pour rompre localement l'étanchéité entre la face extérieure des lèvres d'étanchéité amont 12 et intermédiaire 13 et la face intérieure 15 de la paroi latérale 3 du corps 2.

La figure 2 illustre la seringue 1 en position de stockage (seringue 1 à température ambiante, par exemple dans son emballage). La contenance du corps 2 est adaptée selon le volume souhaité du contenu 18 pour que, dans cette position, le piston 11 soit situé en aval de la rainure 21. Ainsi, l'isolation du contenu 18 (en phase liquide) de la seringue 1 est garantie par les trois lèvres 12, 13, 14 du piston 11. Les chambres 16 et 17 sont étanches et la rainure 21 ne joue aucun rôle.

En début du cycle de stérilisation, la seringue 1 dans son emballage est placée dans l'enceinte de l'autoclave, à température ambiante, la contre pression étant établie. Le contenu 18 de la seringue étant en phase liquide, il n'y a pas de pression qui s'exerce sur le piston 11, tendant à le faire sortir du corps 2 de la seringue 1. De plus, la contre pression régnant dans l'enceinte de l'autoclave s'exerce sur la tige 10 et tend à faire rentrer le piston 11 à l'intérieur du corps 2 de la seringue 1. Le piston 11 est donc toujours dans une position permettant d'isoler le contenu 18.

La température dans l'enceinte de l'autoclave s'élève progressivement jusqu'à atteindre 121°C, soit une pression absolue d'environ 2 bars. Le contenu 18 de la seringue 1 passe alors en phase vapeur, générant ainsi une pression à l'intérieur du corps 2. Cette pression est proportionnelle à la température de la vapeur, et varie aussi en fonction de la quantité de gaz (bulle 19) présent dans le corps 2 de la seringue 1.

Lorsque la pression dans le corps 2 de la seringue 1 génère une force supérieure à celle exercée par la contre pression de l'autoclave sur la tige 10, ajoutée à la force nécessaire au coulissement du piston 11, ce dernier va reculer jusqu'à venir en butée contre le bourrelet 9 (figure 3). La vapeur 23 contenue dans l'enceinte de l'autoclave pénètre alors dans la rainure 21. Compte tenu des relations dimensionnelles mentionnées plus haut, la vapeur 23 pénètre également dans les chambres annulaires 16, 17, permettant ainsi la stérilisation de ces chambres en chaleur dite humide. Dans cette position, l'étanchéité du contenu 18 de la seringue 1 est garantie par la lèvre amont 14 du piston 11. En effet, la rainure 21 est suffisamment longue pour faire communiquer les deux chambres 16, 17 avec l'extérieur du corps 2, et suffisamment courte pour qu'il n'y ait pas de risque de perte d'étanchéité de la chambre intérieure.

Bien entendu, les dimensions du corps 2 de la seringue 1 et le volume du contenu 18 sont prévus pour que, lors de la stérilisation, le piston 11 vienne bien en butée contre le bourrelet 9, et soit donc positionné correctement par rapport à la rainure 21. En outre, la très faible compressibilité du piston 11 permet de garantir l'étanchéité du contenu 18, car la lèvre amont 14 reste à distance de la rainure 21.

En fin de cycle de stérilisation (phase de refroidissement), la pression à l'intérieur du corps 2 de la seringue 1 va diminuer progressivement, et le contenu 18 de la seringue 1 retourner à l'état liquide. Lorsque la contre pression présente dans l'enceinte de l'autoclave générera une force supérieure à celle générée par le contenu de la seringue 1 additionnée de celle nécessaire au coulissement du piston 11, ce dernier va rentrer dans le corps 2 de la seringue 1 et retrouver sa position initiale (figure 2).

Selon un deuxième mode de réalisation, représenté sur les figures 4 et 5, les moyens de passage sont constitués par au moins un orifice 24 pratiqué dans la paroi latérale 3 du corps 2. L'orifice 24, de préférence circulaire et radial, présente un bord amont 25 et un bord aval 26 situés respectivement à la distance $d_{25}$ et $d_{26}$ de la face aval 22 du bourrelet 9, telles que :

$$- \quad d_{25} > h_{12} \quad \text{et} \quad d_{25} < h_{12} + h_{16}$$

$$- \quad d_{26} > h_{12} + h_{16} + h_{13} \quad \text{et} \quad d_{26} < H - h_{14}.$$

Là encore, les dimensions du corps 2 sont adaptées au volume du contenu 18 pour que, en position de stockage (figure 4), le piston 11 soit situé à distance de l'orifice 24, ce dernier n'ayant donc aucun impact sur l'étanchéité de la chambre intérieure. En revanche, l'orifice 24 est agencé pour mettre en communication les deux chambres annulaires 16, 17 avec l'extérieur du corps 2, et ainsi à permettre la pénétration de vapeur 23, lors de la stérilisation (figure 5), lorsque le piston 11 est en butée contre le bourrelet 9.

Enfin, selon un troisième mode de réalisation, les moyens de passage sont constitués par une gorge annulaire 27 ménagée dans la paroi latérale 3 du corps 2, depuis la face intérieure 15. Cette gorge 27 présente une extrémité amont 28 et une extrémité aval 29, et possède les caractéristiques suivantes :

- la distance axiale $d_{28}$ entre la face aval 22 du bourrelet 9 et l'extrémité amont 28 de la gorge 27 est telle que :

$$d_{28} > h_{12} \quad \text{et} \quad d_{28} < h_{12} + h_{16} \text{ ;}$$

- la distance axiale $d_{29}$ entre la face aval 22 du bourrelet 9 et l'extrémité aval 29 de la gorge 27 est telle que : $d_{29} > H$ ;
- la profondeur radiale p' de la gorge 27 est suffisante pour rompre localement l'étanchéité entre la face extérieure des lèvres d'étanchéité intermédiaire 13 et aval 14 et la face intérieure 15 de la paroi latérale 3 du corps 2.
- la longueur axiale de la gorge $(d_{29}-d_{28})$ est inférieure à la longueur axiale totale H du piston 11.

Cette dernière caractéristique permet de garantir l'étanchéité de la chambre intérieure par rapport à l'extérieur du corps 2 de la seringue 1 quelle que soit la position du piston 11 dans le corps 2, entre la position de stockage et la position en butée contre le bourrelet 9.

En variante, la gorge 27 peut s'étendre sur une fraction seulement du pourtour du corps 2.

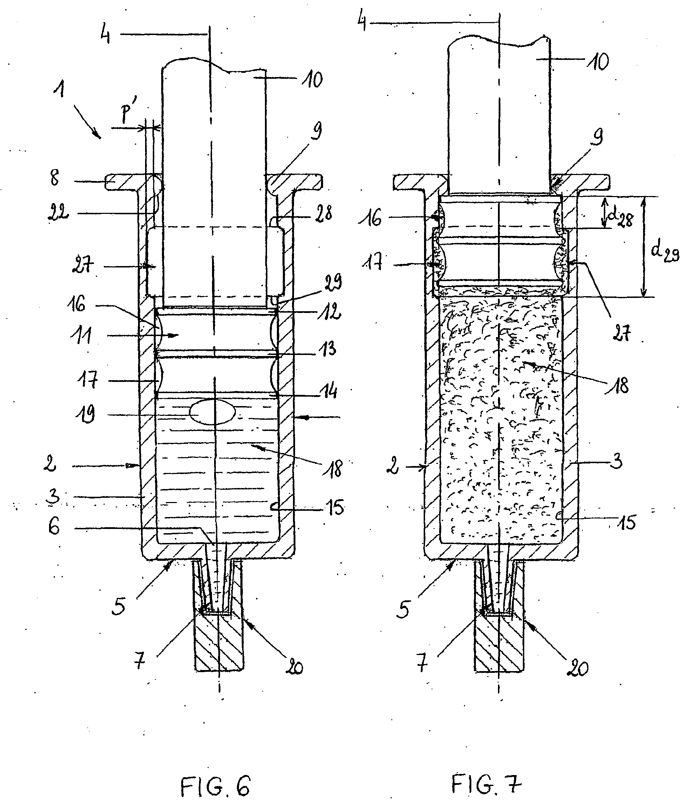

Comme dans les modes de réalisation précédemment décrits, le corps 2 est prévu en fonction du volume du contenu 18 pour que le piston 11 soit situé à distance de la gorge 27 en position de stockage (figure 6) : l'étanchéité de la chambre intérieure n'est alors pas perturbée par la gorge 27.

Lors de la stérilisation (figure 7), le piston 11 vient en butée contre le bourrelet 9, et la gorge 27 met alors en communication l'intérieur du corps 2 avec les chambres annulaires 16, 17. Dans ce mode de réalisation, la vapeur permettant de stériliser les chambres annulaires 16, 17 du piston 11 est formée par le contenu 18, en phase gazeuse, du corps 2 de la seringue 1, et non par la vapeur contenue dans l'enceinte de l'autoclave. Un des avantages de ce mode de réalisation est de permettre la stérilisation de la lèvre aval 14 du piston 11.

Ainsi, par l'adjonction de moyens de passage de la vapeur situés en amont du piston en position de stockage de la seringue et en regard des chambres annulaires du piston lors de la stérilisation, l'invention permet de faire pénétrer la vapeur entre les lèvres du piston, tout en conservant l'isolation du contenu de la seringue de la vapeur de l'enceinte de l'autoclave.

Il va de soi que l'invention n'est pas limitée aux formes de réalisation décrites ci-dessus à titre d'exemples mais qu'elle en embrasse au contraire toutes les variantes de réalisation. Notamment, les moyens de passage pourraient être constitués par une combinaison adaptée des trois modes de réalisation particuliers décrits.

**Revendications**

1. Seringue comprenant :

   - un corps (2) comportant une paroi latérale (3) sensiblement cylindrique présentant une extrémité amont ouverte au voisinage de laquelle est ménagé un bourrelet (9) annulaire intérieur et une extrémité aval fermée par une paroi transversale (5) munie d'un orifice (6) ;
   - une tige (10) formant poussoir, comportant à une extrémité un piston (11) formant joint d'étanchéité possédant au moins deux lèvres d'étanchéité annulaires (12, 13, 14) entre lesquelles est définie au moins une chambre annulaire (16, 17), ladite tige (10) étant apte à être introduite et à coulisser dans le corps (2) ;

   une chambre intérieure destinée à être au moins partiellement remplie d'un contenu (18) étant alors définie entre le piston (11) et la paroi transversale (5) du corps (2) ;
   **caractérisée en ce qu'**elle comprend en outre des moyens de passage (21, 24, 27) ménagés dans le corps (2) de la seringue (1) et agencés pour mettre en communication ladite chambre annulaire ou lesdites chambres annulaires (16, 17) du piston (11) avec l'extérieur du corps (2) ou avec la chambre intérieure du corps (2), lorsque le piston (11) est situé à l'intérieur du corps (2) en butée contre le bourrelet (9) annulaire.

2. Seringue selon la revendication 1, **caractérisée en ce que** les moyens de passage (21, 24, 27) sont agencés pour mettre également en communication au moins une zone de la face extérieure d'au moins une lèvre d'étanchéité (12, 13, 14) avec l'extérieur du corps (2) ou avec la chambre intérieure du corps (2), lorsque le piston (11) est en butée contre le bourrelet annulaire, au moins une autre lèvre d'étanchéité continuant, dans cette position du piston (11), à assurer l'étanchéité de la chambre intérieure du corps (2).

3. Seringue selon la revendication 1 ou 2, **caractérisée en ce que** les moyens de passage comprennent au moins une rainure (21) ménagée sensiblement axialement dans la paroi latérale (3) du corps (2) cylindrique, dans sa face intérieure (15), ladite rainure (21) débouchant hors du corps (2) à l'extrémité amont de celui-ci et s'étendant jusqu'à une extrémité aval située, lorsque le piston (11) est en butée contre le bourrelet annulaire intérieur (9), en amont de la lèvre d'étanchéité aval (14) et en aval de la lèvre d'étanchéité (13) située immédiatement en amont de la lèvre d'étanchéité aval (13), la profondeur radiale (p) de la rainure (21) étant suffisante pour rompre localement l'étanchéité entre la face extérieure d'au moins la lèvre d'étanchéité amont (12) et la face intérieure (15) de la paroi latérale (3) du corps (2).

4. Seringue selon la revendication 1 ou 2, **caractérisée en ce que** les moyens de passage comprennent au moins un orifice (24) pratiqué dans la paroi latérale (3) du corps (2) et agencé pour mettre en communication la chambre annulaire ou les chambres annulaires (16, 17) du piston (11) avec l'extérieur du corps (2) lorsque le piston (11) est en butée contre le bourrelet (9) annulaire, le bord aval (26) dudit orifice (24) étant situé, lorsque le piston (11) est en butée contre le bourrelet annulaire intérieur (9), en amont de la lèvre d'étanchéité aval (14) et en aval de la lèvre d'étanchéité (13) située immédiatement en amont de la lèvre d'étanchéité aval (13).

**5.** Seringue selon la revendication 1 ou 2, **caractérisée en ce que** les moyens de passage comprennent au moins une gorge (27) ménagée dans la paroi latérale (3) du corps (2) cylindrique, dans sa face intérieure (15), ladite gorge (27) s'étendant entre :

- une extrémité amont située, lorsque le piston (11) est en butée contre le bourrelet annulaire intérieur (9), en aval de la lèvre d'étanchéité amont (12) et en amont de la lèvre d'étanchéité (13) située immédiatement en aval de la lèvre d'étanchéité amont (12);
- et une extrémité aval située, lorsque le piston (11) est en butée contre le bourrelet annulaire intérieur (9), en aval de la lèvre d'étanchéité aval (13) ;

la longueur axiale de la gorge (27) étant inférieure à la longueur axiale totale du piston (11) et la profondeur radiale (p') de la gorge (27) étant suffisante pour rompre localement l'étanchéité entre la face extérieure d'au moins la lèvre d'étanchéité aval (14) et la face intérieure (15) de la paroi latérale (3) du corps (2).

**6.** Seringue selon la revendication 5, **caractérisée en ce que** la gorge (27) est annulaire, de même axe (4) que la paroi latérale (3) cylindrique du corps (2).

**7.** Seringue selon l'une des revendications 1 à 6, **caractérisée en ce que** le piston (11) possède trois lèvres d'étanchéité annulaires (12, 13, 14) définissant deux chambres annulaires (16, 17) distinctes, les moyens de passage (21, 24, 27) étant agencés pour mettre en communication chacune des chambres annulaires (16, 17) avec l'extérieur du corps (2) ou avec la chambre intérieure du corps (2) lorsque le piston (11) est situé à l'intérieur du corps (2) en butée contre le bourrelet (9) annulaire.

**8.** Ensemble comportant d'une part un emballage sensiblement imperméable aux bactéries dont au moins une partie est perméable à la vapeur d'eau et d'autre part une seringue (1) selon l'une des revendications précédentes, ladite seringue (1) étant conditionnée dans ledit emballage et sa chambre intérieure au moins partiellement remplie d'un contenu (18).

**Claims**

**1.** A syringe comprising:

- a body (2) having a substantially cylindrical lateral wall (3) having an open upstream end near which an inner annular bead (9) is formed and a downstream end closed by a transverse wall (5) provided with an opening (6);
- a rod (10) forming a plunger, comprising a piston (11) at one end forming a sealing device having at least two annular sealing lips (12, 13, 14) between which at least one annular chamber (16, 17) is defined, said rod (10) being able to be inserted and slide in the body (2);

an inner chamber intended to be at least partially filled with a content (18) then being defined between the piston (11) and the transverse wall (5) of the body (2);
**characterized in that** it also comprises passage means (21, 24, 27) formed in the body (2) of the syringe (1) and arranged to connect said annular chamber(s) (16, 17) of the piston (11) with the outside of the body (2) or with the inner chamber of the body (2), when the piston (11) is situated inside the body (2) abutting against the annular bead (9).

**2.** The syringe according to claim 1, **characterized in that** the passage means (21, 24, 27) are arranged also to connect at least one zone of the outer surface of at least one sealing lip (12, 13, 14) with the outside of the body (2) or with the inner chamber of the body (2), when the piston (11) is abutting against the annular bead, at least one other sealing lip continuing, in this position of the piston (11), to ensure sealing of the inner chamber of the body (2).

**3.** The syringe according to claim 1, **characterized in that** the passage means comprise at least one slot (21) formed substantially axially in the lateral wall (3) of the cylindrical body (2), in its inner surface (15), said slot (21) emerging outside the body (2) at the upstream end thereof and extending to a downstream end situated, when the piston (11) is abutting against the inner annular bead (9), upstream of the downstream sealing lip (14) and downstream of the sealing lip (13) situated immediately upstream of the downstream sealing lip (13), the radial depth (p) of the slot (21) being sufficient to break the sealing locally between the outer surface of at least the upstream sealing lip (12) and the inner surface (15) of the lateral wall (3) of the body (2).

4. The syringe according to claim 1 or 2, **characterized in that** the passage means comprise at least one opening (24) formed in the lateral wall (3) of the body (2) and arranged to connect the annular chamber(s) (16, 17) of the piston (11) with the outside of the body (2) when the piston (11) is abutting against the annular bead (9), the downstream edge (26) of said opening (24) being situated, when the piston (11) is abutting against the inner annular bead (9), upstream of the downstream sealing lip (14) and downstream of the sealing lip (13) situated immediately upstream of the downstream sealing lip (13).

5. The syringe according to claim 1 or 2, **characterized in that** the passage means comprise at least one groove (27) formed in the lateral wall (3) of the cylindrical body (2), in its inner surface (15), said groove (27) extending between:

- an upstream end situated, when the piston (11) is abutting against the inner annular bead (9), downstream of the upstream sealing lip (12) and upstream of the sealing lip (13) situated immediately downstream of the upstream sealing lip (12);
- and a downstream end situated, when the piston (11) is abutting against the inner annular bead (9), downstream of the downstream sealing lip (13);
- the axial length of the groove (27) being smaller than the total axial length of the piston (11) and the radial depth (p') of the groove (27) being sufficient to break the sealing locally between the outer surface of at least the downstream sealing lip (14) and the inner surface (15) of the lateral wall (3) of the body (2).

6. The syringe according to claim 5, **characterized in that** the groove (27) is annular, with the same axis (4) as the cylindrical lateral wall (3) of the body (2).

7. The syringe according to one of claims 1 to 6, **characterized in that** the piston (11) has three annular sealing lips (12, 13, 14) defining two distinct annular chambers (16, 17), the passage means (21, 24, 27) being arranged to connect each of the annular chambers (16, 17) with the outside of the body (2) or with the inner chamber of the body (2) when the piston (11) is situated inside the body (2) abutting against the annular bead (9).

8. An assembly comprising on the one hand a packaging substantially impermeable to bacteria at least part of which is steam-permeable, and on the other hand a syringe (1) according to one of the preceding claims, said syringe (1) being packaged in said packaging and its inner chamber being at least partially filled with content (18).


**Patentansprüche**

1. Spritze, die umfasst:

- einen Körper (2), der eine etwa zylindrische Seitenwand (3) aufweist, der ein offenes oberes Ende aufweist, in dessen Nähe eine ringförmige innere Wulst (9) ausgebildet ist, und ein durch eine Querwand (5) geschlossenes unteres Ende (5), das mit einer Öffnung (6) ausgestattet ist,
- eine Stange (10), die einen Drücker bildet, die an einem Ende einen Kolben (11) aufweist, der eine Dichtung bildet, die mindestens zwei ringförmige Dichtungslippen (12, 13, 14) besitzt, zwischen denen mindestens eine ringförmige Kammer (16, 17) definiert ist, wobei die Stange (10) imstande ist, in den Körper (2) eingeführt zu sein und zu gleiten,

wobei eine innere Kammer dazu bestimmt ist, zumindest teilweise mit einem Inhalt (18) gefüllt zu sein, die dann zwischen dem Kolben (11) und der Querwand (5) des Körpers (2) definiert ist, **dadurch gekennzeichnet, dass** sie ferner Durchgangsmittel (21, 24, 27) umfasst, die in den Körper (2) der Spritze (1) eingearbeitet sind und ausgebildet, um die ringförmige Kammer oder die ringförmigen Kammern (16, 17) des Kolbens (11) mit der Außenwelt des Körpers (2) oder mit der inneren Kammer des Körpers (2) in Kommunikation zu versetzen, wenn sich der Kolben (11) im Körper (2) im Anschlag an der ringförmigen Wulst (9) befindet.

2. Spritze nach Anspruch 1, **dadurch gekennzeichnet, dass** die Durchgangsmittel (21, 24, 27) ausgebildet sind, um ebenfalls mindestens eine Zone der Außenseite mindestens einer Dichtungslippe (12, 13, 14) mit der Außenwelt des Körpers (2) oder mit der inneren Kammer des Körpers (2) in Kommunikation zu versetzen, wenn der Kolben (11) im Anschlag an der ringförmigen Wulst ist, wobei in dieser Stellung des Kolbens (11) mindestens eine andere Dichtungslippe weiterhin die Dichtigkeit der inneren Kammer des Körpers (2) gewährleistet.

3. Spritze nach Anspruch 1 oder 2, **dadurch gekennzeichnet, dass** die Durchgangsmittel mindestens eine Rille (21) umfassen, die etwa axial in die Seitenwand (3) des zylindrischen Körpers (2) auf ihrer Innenseite (15) eingearbeitet ist, wobei die Rille (21) außerhalb des Körpers (2) am oberen Ende desselben mündet und sich bis zu einem unteren Ende erstreckt, das sich, wenn der Kolben (11) im Anschlag an der inneren ringförmigen Wulst (9) ist, über der unteren Dichtungslippe (14) und unter der Dichtungslippe (13) befindet, die sich unmittelbar über der unteren Dichtungslippe (13) befindet, wobei die radiale Tiefe (p) der Rille (21) ausreichend ist, um lokal die Dichtigkeit zwischen der Außenseite mindestens einer oberen Dichtungslippe (12) und der Innenseite (15) der Seitenwand (3) des Körpers (2) zu unterbrechen.

4. Spritze nach Anspruch 1 oder 2, **dadurch gekennzeichnet, dass** die Durchgangsmittel mindestens eine Öffnung (24) umfassen, die in die Seitenwand (3) des Körpers (2) eingearbeitet ist und ausgebildet, um die ringförmige Kammer oder die ringförmigen Kammern (16, 17) des Kolbens (11) mit der Außenwelt des Körpers (2) in Kommunikation zu versetzen, wenn der Kolben (11) im Anschlag an der ringförmigen Wulst (9) ist, wobei sich der untere Rand (26) der Öffnung (24), wenn der Kolben (11) im Anschlag an der inneren ringförmigen Wulst (9) ist, über der unteren Dichtungslippe (14) und unter der Dichtungslippe (13) befindet, die sich unmittelbar über der unteren Dichtungslippe (13) befindet.

5. Spritze nach Anspruch 1 oder 2, **dadurch gekennzeichnet, dass** die Durchgangsmittel mindestens eine Aushöhlung (27) umfassen, die in die Seitenwand (3) des zylindrischen Körpers (2) in ihre Innenseite (15) eingearbeitet ist, wobei sich die Aushöhlung (27) erstreckt zwischen:

   - einem oberen Ende, das sich, wenn der Kolben (11) im Anschlag an der inneren ringförmigen Wulst (9) ist, unter der oberen Dichtungslippe (12) und über der Dichtungslippe (13) befindet, die sich unmittelbar unter der oberen Dichtungslippe (12) befindet,
   - und einem unteren Ende, das sich, wenn der Kolben (11) im Anschlag an der inneren ringförmigen Wulst (9) ist, unter der unteren Dichtungslippe (13) befindet,

   wobei die axiale Länge der Aushöhlung (27) kleiner ist als die axiale Gesamtlänge des Kolbens (11) und die radiale Tiefe (p') der Aushöhlung (27) ausreichend ist, um lokal die Dichtigkeit zwischen der Außenseite mindestens der unteren Dichtungslippe (14) und der Innenseite (15) der Seitenwand (3) des Körpers (2) zu unterbrechen.

6. Spritze nach Anspruch 5, **dadurch gekennzeichnet, dass** die Aushöhlung (27) ringförmig, gleicher Achse (4) wie die zylindrische Seitenwand (3) des Körpers (2) ist.

7. Spritze nach einem der Ansprüche 1 bis 6, **dadurch gekennzeichnet, dass** der Kolben (11) drei ringförmige Dichtungslippen (12, 13, 14) besitzt, die zwei unterschiedliche ringförmige Kammern (16, 17) definieren, wobei die Durchgangsmittel (21, 24, 27) ausgebildet sind, um jede der ringförmigen Kammern (16, 17) mit der Außenwelt des Körpers (2) oder mit der inneren Kammer des Körpers (2) in Kommunikation zu versetzen, wenn sich der Kolben (11) im Körper (2) im Anschlag an der ringförmigen Wulst (9) befindet.

8. Gruppe, die einerseits eine für Bakterien deutlich undurchdringliche Verpackung umfasst, von der mindestens ein Teil für Wasserdampf durchlässig ist und andererseits eine Spritze (1) nach einem der vorangehenden Ansprüche, wobei die Spritze (1) in der Verpackung verpackt ist und ihre innere Kammer mindestens teilweise mit einem Inhalt (18) gefüllt ist.

FIG. 1

FIG. 2

FIG. 3

FIG. 4

FIG. 5

FIG. 6                    FIG. 7

## RÉFÉRENCES CITÉES DANS LA DESCRIPTION

**Documents brevets cités dans la description**

- EP 0599649 A